# EUROPÄISCHE PATENTSCHRIFT

(11) **EP 0 663 381 B1**
(45) Veröffentlichungstag und Bekanntmachung des Hinweises auf die Patenterteilung: **22.04.1998**
(21) Anmeldenummer: 95100049.6
(22) Anmeldetag: 03.01.1995
(51) Int. Cl.: C07C 51/02, C07C 63/36, C07C 65/11, C07C 37/01

(54) **Verfahren zur Freisetzung acider, organischer Verbindungen aus deren Salzen mittels Kohlendioxid**
Process for releasing acidic organic compounds from their salts by means of carbon dioxide
Procédé de libération des composés organiques acidiques de leurs sels à l'aide d'au hydride carbonique

(30) Priorität: 13.01.1994 DE 4400751
(43) Veröffentlichungstag der Anmeldung: 19.07.1995
(73) Patentinhaber: Ticona GmbH, 65926 Frankfurt am Main (DE)
(72) Erfinder: Kulpe, Jürgen, Dr., D-65929 Frankfurt (DE); Strutz, Heinz, Dr., D-61250 Usingen (DE); Rüffer, Hans-Martin, Dr., D-65719 Hofheim (DE); Rittner, Siegbert, Dr., D-64546 Mörfelden (DE)

(56) Entgegenhaltungen:
- EP-A- 0 477 928
- US-A- 3 845 119

## Beschreibung

Die Erfindung betrifft ein Verfahren zur Freisetzung von aciden, organischen Verbindungen aus wäßrigen Lösungen ihrer Salze durch Kohlendioxid in Gegenwart eines in Wasser im wesentlichen unlösbaren organischen Lösungsmittels.

Acide, organische Verbindungen, wie Carbon-, Sulfon- oder Phosphonsäuren, sind in allen Bereichen der organischen Chemie von Bedeutung. Beispielsweise sind aromatische Carbonsäuren und auch aromatische Hydroxyverbindungen Ausgangsverbindungen für eine Vielzahl industrieller Anwendungen, wie die Herstellung von Polyestern oder Polyamiden. Auch aus Hydroxycarbonsäuren lassen sich durch Kondensation mit sich selbst Polyester herstellen. Aromatische Sulfonsäuren sind vielseitig verwendbare Zwischenprodukte, die z. B. in der Farbstoff- und Pigmentherstellung Verwendung finden.

Die Herstellung von aromatischen Hydroxyverbindungen, Carbonsäuren und Sulfonsäuren ist Stand der Technik. Häufig wird dabei eine alkalische Reaktionsphase durchlaufen, die einen Neutralisations- bzw. Ansäuerungsschritt zur Freisetzung der Produkte notwendig macht. Gleiches gilt für aromatische Hydroxycarbonsäuren, die in der Regel ausgehend von den Hydroxyverbindungen durch die sogenannte Kolbe-Schmitt-Reaktion hergestellt werden. Hierbei wird das Alkalimetallsalz der entsprechenden Hydroxyverbindung bei erhöhter Temperatur mit Kohlendioxid zur Reaktion gebracht. Dabei entsteht das Alkalimetallsalz der entsprechenden Hydroxycarbonsäure. Es kann durch Zusatz einer Mineralsäure in Freiheit gesetzt und isoliert werden. Hierbei entstehen pro Grammatom Hydroxycarbonsäure zwei Grammatome des Alkalisalzes der verwendeten Mineralsäure. Die Alkalisalze der Mineralsäuren können für weitere Reaktionen nicht mehr verwendet werden und stellen somit einen Zwangsanfall von Salz dar, welcher entsorgt werden muß.

Wünschenswert ist die Vermeidung dieses ökologisch und ökonomisch ungünstigen Zwangsanfalls von Salz bei der Freisetzung der aromatischen, aciden Verbindungen aus ihren Metallsalzen.

In einer Reihe von Verfahren wird anstelle von Mineralsäuren Kohlendioxid, als gasförmiges Säureanhydrid der Kohlensäure, zur Freisetzung von organischen Säuren eingesetzt. Der Vorteil dieser Vorgehensweise liegt in der Tatsache, daß die Kohlensäure im Gegensatz zu Mineralsäuren leicht aus ihren Metallsalzen wieder freigesetzt werden kann, wobei die Metallbase als Wertstoff zurückgewonnen werden kann. Dies gelingt teilweise bereits durch Erhöhung der Temperatur unter Bildung der Metalloxide.

Problematisch bei der Verwendung von Kohlendioxid als Anhydrid der Kohlensäure bei Säure-Base-Reaktionen ist die Existenz einer scheinbaren Dissoziationskonstante (pK_{S}). Diese scheinbare Dissoziationskonstante charakterisiert die abgeschwächte Säurestärke einer wäßrigen Kohlensäurelösung. Die scheinbare Dissoziationskonstante setzt sich aus der tatsächlichen Dissoziationskonstante der ersten Stufe der Kohlensäure und dem vorgelagerten Hydrationsgleichgewicht von physikalisch gelöstem Kohlendioxid zu Kohlensäure zusammen. Diese scheinbare Dissoziationskonstante bewirkt, daß eine Säure, die sich laut Vergleich ihrer Dissoziationskonstante mit der ersten Dissoziationskonstante der Kohlensäure in Freiheit setzen lassen sollte, nicht merklich freisetzen läßt. In der Praxis muß somit mit der scheinbaren Dissoziationskonstante verglichen werden, wodurch sich nur noch eine geringe Anzahl acider, organischer Verbindungen durch Kohlensäure vollständig aus ihren Salzen in Freiheit setzen lassen. Bei Nichtgleichgewichtsreaktionen ist beschrieben (Ang. Chem. 103 (1991), 1689), daß durch Erhöhung des Drucks die Einstellung des Hydratationsgleichgewichts beschleunigt werden kann.

Damit werden vollständige Umsetzungen möglich, wenn das durch Einwirkung von Kohlendioxid als Säure gebildete Produkt abreagiert und damit aus dem Gleichgewicht entfernt wird.

Bei der Reaktion von Kohlensäure mit Alkalisalzen organischer Säuren werden die Alkalihydrogencarbonate gebildet, die in wäßriger Lösung den pH-Wert puffern und damit den minimal erreichbaren pH-Wert limitieren. In der JP-A 50/01099 wird dies umgangen, indem der Lösung ein wassermischbares organisches Lösungsmittel zugesetzt wird, das die Löslichkeit des gebildeten Hydrogencarbonates reduziert. Hierdurch fällt das Hydrogencarbonat aus und ist somit aus dem Gleichgewicht entfernt. Nachteil ist, daß die Abtrennung des Kaliumhydrogencarbonats nur zu ca. 80 % gelingt.
Die Isolierung organischer Säuren aus ihren Salzen mittels Kohlendioxid durch Zusatz eines wassernichtmischbaren Kohlenwasserstoffes ist in der JP-A 55/00322 beschrieben. Nachteil dieses Verfahrens ist, daß die organische Säure in dem Kohlenwasserstoff hinreichend löslich sein muß, was nur in Ausnahmen der Fall ist.

In US-A 4,282,323 ist das Entfernen und Konzentrieren von niedermolekularen organischen Säuren aus wäßrigen Fermentationslösungen durch Ansäuern mit Kohlendioxid und Extraktion der Säure mit einem polaren organischen Lösungsmittel, wie t-Butanol, beschrieben.

Nachteile des Verfahrens sind die geringe Ausbeute und die Verunreinigung der erhaltenen Säure mit Salzen. Dieses Verfahren ist prinzipiell daher nur für solche Säuren gut geeignet, die sich destillativ einfach abtrennen und reinigen lassen.

Es wurde nun überraschend gefunden, daß sich acide, organische Verbindungen in hoher Reinheit und guter Ausbeute erhalten lassen, wenn das zur Extraktion der freien Säure aus der wäßrigen Phase verwendete polare organische Lösungsmittel unter Kohlendioxiddruck mit Wasser reextrahiert wird.

Gegenstand der Erfindung ist ein Verfahren zur Freisetzung von aciden organischen Verbindungen aus wäßrigen Lösungen ihrer Salze durch Kohlendioxid in Gegenwart eines im wesentlichen wasserunlöslichen organischen Lösungsmittels, dadurch gekennzeichnet, daß die organische Phase mit kohlendioxidhaltigem Wasser reextrahiert wird.

Bei dem erfindungsgemäßen Verfahren werden die aciden Verbindungen vollständig aus den entsprechenden Salzen in Freiheit gesetzt, d. h. die organische Lösung ist frei von Salzen.

Die Produkte können aus der organische Lösung auf den üblichen Wegen, z. B. Eindampfen oder Kristallisation isoliert werden. Die in der wäßrigen Phase zurückgehaltenen Metallsalze der Kohlensäure lassen sich entweder direkt oder nach Eindampfen, Trocknen und gegebenenfalls Calcinieren als Einsatzbase in den Prozeß zurückführen oder auch anderweitig vielseitig verwerten. Von ökologisch und ökonomisch besonderer Bedeutung ist, daß die verwendeten Lösungsmittel vollständig im Kreis geführt werden können.

Acide, organische Verbindungen im Sinne der Erfindung sind alle organischen Verbindungen, die acide, durch Metalle ersetzbare Protonen enthalten. Beispiele sind Carbonsäuren, Sulfonsäuren, Phosphonsäuren, aber auch Phenole, Naphthole und aliphatische Alkohole.

Bevorzugt sind acide, organische Verbindungen mit einem pK_{S}-Wert von 2 bis 10, besonders bevorzugt von 3 bis 6. Ganz besonders bevorzugt werden mit dem erfindungsgemäßen Verfahren Carbon-, Hydroxycarbon- und Aminosäuren aus ihren Salzen freigesetzt, insbesondere 6-Hydroxy-2-napthalincarbonsäure, 3-Hydroxy-2-naphthalincarbonsäure, Salicylsäure, Parahydroxybenzoesäure und Ph-SO₂-N(CH₃)-(CH₂)₅-COOH.

Als Ausgangsverbindungen in dem erfindungsgemäßen Verfahren werden im allgemeinen Metallsalze der entsprechenden aciden, organischen Verbindungen eingesetzt. Bevorzugt sind Alkali- und Erdalkalimetallsalze, besonders bevorzugt Lithium-, Natrium- und Kaliumsalze, ganz besonders bevorzugt Natrium- und Kaliumsalze.

Die zum Einsatz im erfindungsgemäßen Verfahren geeigneten Salze acider, organischer Verbindungen können auch in geringer Menge im Gemisch mit einer großen Menge anderer Salze vorliegen. Solche Salzgemische fallen z. B. bei Kolbe-Schmitt-Reaktionen und verwandten Reaktionen an, wie sie beispielsweise in GB-A 1 155 176 und US-A 3 655 744 beschrieben sind.

Beispielhaft sei die Herstellung von 6-Hydroxy-2-naphthalicarbonsäure, im weiteren als HNA bezeichnet, genannt. Bei der Herstellung von HNA fällt ein Reaktionsgemisch an, das neben dem Dikaliumsalz der HNA aus 3-Hydroxy-2-naphthalincarbonsäure (BONS), dem Dikaliumsalz von BONS, Kalium-2-naphtholat, dem Kaliumsalz der 6-Hydroxy-2,7-naphthalindicarbonsäure (HNDA), Kaliumcarbonat und Kaliumformiat besteht.

Ebenso geeignet sind Fermentationslösungen, wie sie z. B. in US-A 4,282,323 beschrieben sind.

Die eingesetzten Metallsalze werden mit Kohlendioxid zu den entsprechenden freien aciden, organischen Verbindungen und Metallhydrogencarbonaten umgesetzt. Im allgemeinen wird unter Kohlendioxiddruck gearbeitet, vorzugsweise bei Drücken von 1 bis 70 bar, besonders bevorzugt von 1 bis 20 bar, ganz besonders bevorzugt von 2 bis 6 bar.

Die Zufuhr des Kohlendioxids kann beispielsweise über eine feststehende Begasungseinrichtung, einen Hohlschaftbegasungsrührer, über die Flüssigkeitsoberfläche oder durch eine Vorsättigung einer oder beider Flüssigkeitsphasen erfolgen.

Die beim Ansäuern mit Kohlendioxid freigesetzte acide, organische Verbindung wird durch Extraktion mit einem im wesentlichen wasserunlöslichen organischen Lösungsmittel aus dem Reaktionsgleichgewicht entfernt.

Im wesentlichen wasserunlöslich bedeutet im Sinne der Erfindung, daß sich unter den gewählten Extraktionsbedingungen zwei, durch eine Phasengrenze getrennte Phasen ausbilden.

Geeignet sind polare organische Lösungsmittel, wie Alkohole, Ketone, Ester und Ether. Bevorzugte Lösungsmittel sind 1-Butanol, höhere Alkohole, sekundäre und tertiäre Alkohole. Im Falle der HNA hat sich 1-Butanol als besonders günstiges Extraktionsmittel erwiesen.

Die Extraktion kann kontinuierlich oder diskontinuierlich erfolgen. Alle Apparate, die die geforderten Bedingungen für die Extraktion aufrechterhalten können, sind für das erfindungsgemäße Verfahren geeignet. Bevorzugt sind kontinuierlich und mehrstufig arbeitende Flüssig-flüssig-Extraktoren im Gegenstrombetrieb, z. B. Mixer-Settler, Zentrifugalextraktoren und Kolonnenextraktoren gemäß dem Stand der Technik (siehe z. B. Ullmann's Encyclopaedia of Industrial Chemistry, Vol. B3, 1988).

Das erfindungsgemäße Verfahren wird im allgemeinen bei Temperaturen zwischen dem Festpunkt der Lösung und 150°C, vorzugsweise von -10 bis 100°C, besonders bevorzugt von 10 bis 60°C, durchgeführt.

Erfindungsgemäß wird die bei der obigen Extraktion erhaltene organische Phase mit kohlendioxidhaltigem Wasser reextrahiert.

Dabei wird im allgemeinen unter Kohlendioxiddruck, vorzugsweise von 1 bis 70 bar, besonders bevorzugt von 1 bis 20 bar, ganz besonders bevorzugt von 2 bis 6 bar gearbeitet.

Die Reextraktion wird im allgemeinen bei einer Temperatur zwischen dem Festpunkt der Lösung und 150°C, vorzugsweise von -10 bis 100°C, besonders bevorzugt von 10 bis 60°C, durchgeführt.

Sie kann kontinuierlich oder diskontinuierlich betrieben werden. Für die kontinuierliche Reextraktion sind beispielsweise Mixer-Settler oder im Gegenstrom betriebene Extraktionskolonnen geeignet.

Zur Durchführung des erfindungsgemäßen Verfahrens sind alle Gefäße, die zum Betrieb unter den genannten Temperatur- und Druckbedingungen geeignet sind, verwendbar.

Im Folgenden ist ein möglicher Weg zur Durchführung des erfindungsgemäßen Verfahrens am Beispiel der 6,2 HNA beschrieben:

Zunächst wird das wäßrige anfallende oder in Wasser gelöste Salz oder Salzgemisch (im Falle von HNA : HNA-Dikaliumsalz, 3-Hydroxy-2-naphtalincarbonsäure-dikaliumsalz, Kalium-2-naphtholat, HNDA-Trikaliumsalz, Kaliumcarbonat und Kaliumformiat) mit dem im wesentlichen wasserunlöslichen organischen Lösungsmittel versetzt und die Phasen werden durch Rühren innig vermischt. Dann wird unter Druck mit Kohlendioxid solange angesäuert, bis der pH-Wert konstant ist. Der pH-Wert kann beispielsweise mit einer handelsüblichen, druckstabilen Glaselektrode gemessen werden.

Nachdem ein stabiler pH-Wert erreicht ist, trennt man die organische Phase ab, vorzugsweise unter Druck, und wiederholt die Extraktion, im Falle der diskontinuierlichen Aufarbeitung, solange noch Zielverbindung in der wäßrigen Phase enthalten ist. Die wäßrige Phase kann das organische Lösungsmittel (im Falle von HNA vorzugsweise 1-Butanol) in Sättigungskonzentration enthalten. Dieses kann destillativ, häufig unter Ausnutzung von Azeotropen, zurückgewonnen werden. Die dann verbleibende Salzlösung kann in der Synthese wieder eingesetzt werden.

Die organischen Extrakte werden gegebenenfalls, bei diskontinuierlicher Verfahrensführung, vereinigt. Sie enthalten im Falle von HNA neben 1-Butanol, Wasser, Kaliumformiat, in Spuren Kaliumhydrogencarbonat, freies Naphthol sowie HNA und BONS mit 1 bis 1,5 Kalium pro Molekül.

Die Lösung wird mit kohlensaurem Wasser reextrahiert. Hierbei wird vorteilhafterweise kräftig gerührt oder eine andere geeignete Maßnahme ergriffen, um die beiden Phasen innig miteinander zu vermischen. Wenn der pH-Wert sich nicht mehr ändert, werden organische und wäßrige Phasen getrennt. Die Prozedur wird gegebenenfalls, bei diskontinuierlicher Verfahrensführung, solange wiederholt, bis der Salzgehalt der organischen Phase die gewünschten Werte erreicht hat.

Die bei dieser Extraktion anfallende Wasserphase kann geringe Mengen des organischen Lösungsmittels, ferner die Metallanteile aus der organischen Phase in Form des Hydrogencarbonates enthalten. Jedoch wird auch ein Teil der in Freiheit gesetzten, sauren, organischen Verbindungen mit in die Wasserphase extrahiert. Um diesen Anteil nicht zu verlieren, kann die Wasserphase zum Beginn des Verfahrens geführt, zur Auflösung des Salzgemisches verwendet und damit in den Kreislauf zurückgeführt werden.

Liegen in der wäßrigen Lösung die Salze verschiedener acider, organischer Verbindungen mit unterschiedlichen pK_{S}-Werten vor, kann nach dem erfindungsgemäßen Verfahren auch eine Trennung dieser Verbindungen durch selektives Freisetzen einer Verbindung erfolgen. Diese kann beispielsweise durch Fällung oder Extraktion abgetrennt werden, wobei die Extraktion bevorzugt ist. Anschließend kann durch weitere Zufuhr die stärker acide Verbindung aus ihrem Salz freigesetzt und isoliert werden. Im Falle von HNA ist es beispielsweise möglich, zuerst selektiv β-Naphthol, das als K-β-Naphtholat vorliegt, und anschließend HNA selbst abzutrennen.

Ferner gelingt es bei HNA, die als Nebenkomponente bei der Herstellung gebildete 6-Hydroxy-2,7-naphthalindicarbonsäure (HNDA) durch Filtration nach der HNA-Extraktion abzutrennen, indem die Extraktionsbedingungen so gewählt werden, daß nur weniger als 1 % der HNDA extrahiert werden.

Die erfindungsgemäß hergestellten freien aciden, organischen Verbindungen sind im allgemeinen so rein, daß sie ohne weitere Reinigungsschritte weiter umgesetzt oder verwendet werden können. Werden höhere Reinheiten gewünscht, können diese durch Umkristallisation, im Fall der HNA wiederum aus Butanol, erzielt werden. Insbesondere kann erfindungsgemäß hergestellte HNA zu einem Polyester umgesetzt werden, der z. B. unter der Bezeichnung Vectra® von der Hoechst Celanese Corporation, USA, vertrieben wird. Die Erfindung wird durch die Beispiele näher erläutert, ohne sie darauf beschränken zu wollen.

In den Beispielen besteht das Reaktionsgefäß aus einem Autoklav mit 2 Liter Innenvolumen, der über einen Doppelmantel temperiert ist. Der Autoklav ist mit einer Pumpe zur Flüssigkeitsdosierung, Innenthermometer, druckstabiler pH-Elektrode, Tauchrohr und Hohlschaftsbegasungsrührer versehen, der in der Hälfte seiner Gesamtlänge mit einer Rührscheibe versehen ist. Der Rührer ist magnetisch an den Antrieb gekuppelt. Zur pH-Wert-Messung wird eine druckstabile Elektrode der Firma Ingold, Frankfurt, Typ Infit® 764-50 verwendet. Bei pH-Wert-Messungen wird die Temperatur manuell kompensiert.

Der Gehalt an organischen Verbindungen sowie an Carbonat- und Formiationen wurde mit Hochdruckflüssigkeitschromatographie (HPLC), der Kaliumgehalt mittels AAS-Spektroskopie bestimmt. Bei Feststoffen wurde der Wassergehalt nach Karl-Fischer bestimmt. Eine Differenzierung zwischen Carbonat- und Hydrogencarbonat-Ionen war nicht möglich. Die Nachweisgrenzen für Formiat-und Carbonationen lag bei 0,01 % (m/m).

### Beispiele:

### 1. Freisetzung von 6-Hydroxy-2-naphthalincarbonsäure (HNA) aus ihrem Dikaliumsalz

Eine Lösung, bestehend aus 17,8 g HNA, 12,77 g Kaliumhydroxid, 13,1 g Kaliumcarbonat, 237 g Kaliumformiat und 350 ml Wasser, wird im Autoklav auf 50°C erwärmt. Zu der wäßrigen Lösung werden 500 ml 1-Butanol gegeben und das Zweiphasensystem kräftig gerührt. Dann wird im Gasraum des Reaktors ein Kohlendioxid-Druck von 5 bar eingestellt. Nach einer Stunde wird die Rührung ausgeschaltet, worauf sich zwei flüssige Phasen ausbilden. Über das Tauchrohr werden 470 ml wasserhaltige Butanol-Phase abgetrennt und nach Entleerung und Spülung des Reaktors in diesen zurückgepumpt. Anschließend werden 670 ml Wasser nachgepumpt. Nach 30 Minuten wird die Rührung unterbrochen, organische und wäßrige Phase werden getrennt und die organische Phase in den Reaktor zurückgepumpt. Die Reextraktion wird mit 660 ml Wasser wiederholt. Nach der Phasentrennung werden 365 ml Butanol-Phase isoliert. Das Lösungsmittel wird im Vakuum abgedampft und der erhaltene Rückstand bei 50°C im Vakuum < 20 mbar über Nacht getrocknet.
Isoliert werden 9,26 g HNA als rein weißes Pulver.
Wassergehalt: 0,024 %; Schmelzpunkt: 247 bis 248°C; das Produkt enthält keine mittels HPLC-Analyse nachweisbaren Mengen an Carbonat und Formiat.

| Elementaranalyse: | | | |
|---|---|---|---|
| berechnet: | C 70,21 | H 4,28 | K 0 |
| gefunden: | C 70,0 | H 4,15 | K 0,048 |

### 2. Freisetzung von 3-Hydroxy-2-naphthalincarbonsäure (BONS) aus ihrem Dinatriumsalz

Es wird die wäßrige Lösung einer Kolbe-Schmitt-Reaktion, wie sie bei der Herstellung von BONS anfällt, eingesetzt. Die Lösung enthält Natrium-2-naphtholat, Natriumcarbonat, Natriumhydroxid, 6-Hydroxy-2-naphthalincarbonsäure sowie 3-Hydroxy-2-naphthoesäuredinatriumsalz. Die Lösung hat einen pH-Wert von 13,35. Es werden 800 ml (≙ 837 g) der Lösung in den Reaktor gefüllt und auf 50°C aufgeheizt. Dann wird durch vorsichtige Kohlendioxid-Zugabe der pH-Wert innerhalb von 10 Minuten auf einen pH-Wert von 9 abgesenkt. Dabei scheiden sich 7,5 g eines aus 2-Naphthol und Harzen bestehenden Feststoffes ab, die durch Filtration abgetrennt werden. Nach Zugabe von 500 ml 1-Butanol wird ein Kohlendioxiddruck von 5,45 bar in der Gasphase eingestellt. Nach 75 Minuten wird die Rührung unterbrochen und die Phasen getrennt. Die organische Phase wird anschließend bei 25°C und 5,5 bar Kohlendioxid-Druck dreimal mit je 220 ml Wasser reextrahiert. Aus der Butanol-Phase werden, wie in Beispiel 1 beschrieben, 13,7 g eines bräunlich-gelben Pulvers isoliert, das aus einem Gemisch von BONS, 2-Naphthol sowie < 1 % HNA besteht. Der Natriumgehalt des Produktgemisches ist < 1 %.

### Beispiel 3: Abtrennung von 2-Naphthol durch selektive Ansäuerung in Gegenwart eines geeigneten Lösungsmittels

Lösung analog Beispiel 2, jedoch wird die Reaktion bei 42°C durchgeführt. Vor der Ansäuerung werden 150 ml Methyl-(tertiär-butyl)-ether zugegeben. Der pH-Wert wird auf 7,75 abgesenkt. Die Produktisolierung erfolgt durch Abdampfen des Lösungsmittels im Vakuum und Trocknung des isolierten Feststoffes bei 40°C bei einem Druck von 20 mbar über Nacht. Isoliert werden 6,4 g eines bräunlichen Pulvers, das aus 7,3 % HNA, 0,2 % BONS und 91,8 % 2-Naphthol besteht. Bezogen auf eingesetztes Material werden 94,8 % des 2-Naphthols isoliert.

### Vergleichsversuch: Abtrennung durch pH-selektive Fällung von 2-Naphthol ohne organisches Lösungsmittel

Eine Lösung aus 25,7 g Kaliumhydroxid 84,2 %ig, 6,2 g Naphthol, 35,3 g 6,2-HNA, 2,7 g BONS, 16,7 g Kaliumcarbonat und 45 g Kaliumformiat in 640 ml Wasser wird im Reaktor auf 50°C erwärmt. Durch Kohlendioxid-Zugabe wird der pH-Wert auf 8,0 abgesenkt. Dabei fällt in der Lösung ein hellbrauner Feststoff aus. Der Feststoff wird abgesaugt, mit Wasser gewaschen und getrocknet. Isoliert wurden 4,75 g eines hellbraunen Pulvers, das aus 2,2 % HNA, 0,9 % BONS und 93 % 2-Naphthol besteht. Dies sind 71 % der Theorie bezogen auf isoliertes 2-Naphthol.

### Beispiel 4: Freisetzung von 1-Naphtalincarbonsäure aus ihrem Kaliumsalz

Eine alkalische Lösung bestehend aus 20 g 1-Naphthalincarbonsäure und 10 g Kaliumhydroxid in 500 ml Wasser wurde in einem 2 Liter Edelstahlautoklav mit 500 ml 1-Butanol versetzt und bei konstanter Drucküberlagerung mit Kohlendioxid von 5,0 bar bei Raumtemperatur kräftig gerührt. Der Autoklav verfügt dabei über einen selbstansaugenden Hohlwellenrührer zur Gasverteilung in die Emulsion. Nach einer Stunde wird der Rührer ausgeschaltet und den beiden Phasen Gelegenheit zur Trennung gegeben. Beide Phasen werden mit Hilfe eines Tauchrohres noch unter Druck getrennt entnommen, wobei 488 g leichte Phase und 411 g schwere Phase erhalten werden. Die leichte Phase wird komplett zusammen mit 1000 ml Wasser wie in Beispiel 1 beschrieben einer einfachen Reextraktion unterzogen. Hierbei werden 355 g leichte Phase isoliert, die nach Abdestillieren des Lösungsmittels 18,72 g des Produktes in trockener Form mit einem Kaliumgehalt unter 0,4 % enthalten.
gefunden:
- Ausbeute:: 94 %
- Ansäuerung:: 99 %

### Beispiel 5: Freisetzung von Benzoesäure aus ihrem Kaliumsalz

Eine alkalische Lösung bestehend aus 20 g Benzoesäure und 20 g Kaliumhydroxid in 500 ml Wasser wurde in derselben Weise wie Beispiel 4 behandelt. Die nach einfacher Reextraktion erhaltene leichte Phase von 366 g enthält nach dem Abdestillieren des Lösungsmittels 23,32 g des Produktes in trockener Form mit einem Kaliumgehalt von 1,2 %.
gefunden:
- Ausbeute:: 29 %
- Ansäuerung:: 96 %

### Beispiel 6: Freisetzung von 1-Naphthalinsulfonsäure aus ihrem Kaliumsalz

Eine alkalische Lösung bestehend aus 40 g 1-Naphthalinsulfonsäure und 40 g Kaliumhydroxid in 500 ml Wasser wird in derselben Weise wie Beispiel 4 behandelt. Die nach einfacher Reextraktion erhaltene leichte Phase von 366 g enthält nach dem Abdestillieren des Lösungsmittels 2,32 g des Produktes in trockener Form mit einem Kaliumgehalt von 7,9 %.
gefunden:
- Ausbeute:: 5 %
- Ansäuerung:: 55 %

### Beispiel 7: Freisetzung von

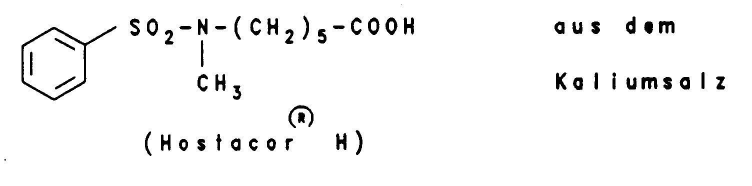

20 g Hostacor® werden in 500 ml E-Wasser mit 12,5 g Kalilauge (50 %) alkalisiert und gelöst. Die Lösung wird in einem mit einem schnellrührenden Hohlrührer ausgestatteten 2-l Autoklav zusammen mit 500 ml Butanol 1 h lang unter 4 bar CO₂ kräftig gerührt. Nach Trennung der Phasen wird die Butanolphase anschließend dreimal mit 500 ml entmineralisiertem Wasser unter gleichen Bedingungen reextrahiert. Die verbliebene Butanolphase (360 g) wird eingedampft und der Rückstand im Trockenschrank getrocknet.
- Ausbeute:: 70 %
- Ansäuerung:: 95 %
(Kaliumgehalt = 0,04 %)

### Beispiel 8: Wiedereinsatz des Reextraktionswassers zur Auflösung des Salzgemisches

A. Eine Lösung von 25,7 g Kaliumhydroxid (84,2 %ig), 6,2 g 2-Naphthol, 2,8 g 3-Hydroxy-2-naphthoesäure, 35,3 g 6-Hydroxy-2-naphthoesäure, 16,7 g Kaliumcarbonat und 45 g Kaliumformiat in 670 ml Wasser wird im Autoklav auf 50°C erwärmt und schubweise mit Kohlendioxid versetzt, bis die Lösung einen pH-Wert von 8 aufweist. Die Lösung wird über eine Drucknutsche filtriert. Dabei werden 4,35 g eines hellbraunen Feststoffes isoliert, der zu mehr als 89 % aus 2-Naphthol (63 % der Theorie), sowie aus 2,3 % HNA (0,3 % der Theorie) besteht. Das Filtrat wird in den Autoklav zurückgeführt, mit 400 ml wäßrigem Butanol versetzt, bei 24°C mit 5,3 bar Kohlendioxiddruck überlagert und kräftig gerührt. Wenn sich der pH-Wert nicht mehr ändert, wird die Rührung unterbrochen und die organische Phase abgetrennt. Die Extraktion wird anschließend dreimal mit je 250 ml wäßrigem Butanol wiederholt. Die organischen Phasen werden vereinigt und viermal mit je 200 ml Wasser unter 5,3 bar Kohlendioxiddruck reextrahiert. Danach werden 1053 g Butanol-Phase isoliert. Das Lösungsmittel wird im Vakuum abgedampft und der erhaltene Rückstand bei 50°C im Vakuum < 20 mbar über Nacht getrocknet. Isoliert werden 31,7 g eines hellbraunen Pulvers, das aus 88,2 % HNA (79 % der Theorie), 7,2 % BONS (81 % der Theorie), sowie 5,3 % 2-Naphthol (27 % der Theorie) besteht. Der Kaliumgehalt liegt unter 0,8 %. Die bei der Reextraktion isolierten schwereren, wasserreichen Phasen werden vereinigt. Durch Destillation des Azeotrops wird das Butanol entfernt, wobei 700 ml Wasserphase erhalten werden.
B. Das Beispiel 8 A wird wiederholt, wobei zum Auflösen des Kaliumhydroxids, Naphthols, 3-Hydroxy-2-naphthoesäure, 6-Hydroxy-2-naphthoesäure, Kaliumcarbonats und Kaliumformiats die in Beispiel 8 A erhaltenen, vom Butanol befreiten 700 ml Rückextraktionswasser eingesetzt werden. Durch Aufarbeitung der reextrahierten Butanolphase werden 36,9 g eines hellbraunen Pulvers isoliert. Das Pulver besteht zu 88,5 % aus HNA, dies entspricht 92,5 % der in dem Versuch eingesetzten HNA-Menge, ferner zu 6,45 % aus BONS (85 % der Theorie) und zu 5,1 % aus Naphthol, (30 % der Theorie).

### Beispiel 9: Abtrennung von 6-Hydroxy-2,7-naphthalindicarbonsäure

Löst man zusätzlich zu den in Beispiel 8 A beschriebenen Menge 5,4 g 6-Hydroxy-2,7-naphthalindicarbonsäure (HNDA) und 5,9 g Kaliumhydroxid und arbeitet anschließend wie beschrieben weiter, findet man nur 1,1 % der eingesetzten HNDA-Menge im Rückstand der Butanolphase. Mehr als 80 % der eingesetzten HNDA-Menge können durch Filtration der nach der Butanol-Extraktion verbleibenden Salzlösung isoliert werden. In der Lösung selbst verbleiben ca. 3 % der HNDA-Einsatzmenge.

## Patentansprüche

1. Verfahren zur Freisetzung von aciden organischen Verbindungen aus wäßrigen Lösungen ihrer Salze durch Kohlendioxid in Gegenwart eines in Wasser im wesentlichen unlösbaren organischen Lösungsmittels, dadurch gekennzeichnet, daß die organische Phase mit kohlendioxidhaltigem Wasser reextrahiert wird.

2. Verfahren nach Anspruch 1, dadurch gekennzeichnet, daß bei Extraktion und/oder Reextraktion unter Kohlendioxiddruck gearbeitet wird.

3. Verfahren nach Anspruch 1 oder 2, dadurch gekennzeichnet, daß der Kohlendioxiddruck zwischen 1 und 70 bar liegt.

4. Verfahren nach Anspruch 3, dadurch gekennzeichnet, daß die Extraktions- und Reextraktionstemperatur zwischen dem Festpunkt der Lösung und 150°C liegt.

5. Verfahren nach einem oder mehreren der Ansprüche 1 bis 4, dadurch gekennzeichnet, daß das organische Lösungsmittel ausgewählt ist aus der Gruppe Alkohole, Ester, Ketone und Ether.

6. Verfahren nach einem oder mehreren der Ansprüche 1 bis 5, dadurch gekennzeichnet, daß es kontinuierlich durchgeführt wird.

7. Verfahren nach einem oder mehreren der Ansprüche 1 bis 5, dadurch gekennzeichnet, daß Alkali- oder Erdalkalimetallsalze der freizusetzenden aciden, organischen Verbindung eingesetzt werden.

8. Verfahren nach einem oder mehreren der Ansprüche 1 bis 7, dadurch gekennzeichnet, daß eine selektive Trennung von aciden, organischen Verbindungen durch Extraktion erfolgt.

9. Verfahren nach einem oder mehreren der Ansprüche 1 bis 8, dadurch gekennzeichnet, daß ein Salz oder Salze der 6-Hydroxy-2-naphthalincarbonsäure eingesetzt werden.

## Claims

1. A process for releasing acidic organic compounds from aqueous solutions of their salts by carbon dioxide in the presence of an essentially water-insoluble organic solvent, which comprises re-extracting the organic phase with carbon dioxide-containing water.

2. The process as claimed in claim 1, wherein carbon dioxide pressure is employed for extraction and/or re-extraction.

3. The process as claimed in claim 1 or 2, wherein the carbon dioxide pressure is between 1 and 70 bar.

4. The process as claimed in claim 3, wherein the extraction and re-extraction temperature is between the solid point of the solution and 150°C.

5. The process as claimed in one or more of claims 1 to 4, wherein the organic solvent is selected from the group consisting of alcohols, esters, ketones and ethers.

6. The process as claimed in one or more of claims 1 to 5, wherein it is carried out continuously.

7. The process as claimed in one or more of claims 1 to 5, wherein alkali metal salts or alkaline earth metal salts of the acidic organic compound to be released are used.

8. The process as claimed in one or more of claims 1 to 7, wherein selective separation of acidic organic compounds is performed by extraction.

9. The process as claimed in one or more of claims 1 to 8, wherein a salt or salts of 6-hydroxy-2-naphthalenecarboxylic acid are used.

## Revendications

1. Procédé pour libérer des composés organiques acides à partir de solutions aqueuses de leurs sels à l'aide du dioxyde de carbone en présence d'un solvant organique essentiellement insoluble dans l'eau, caractérisé en ce que l'on réextrait la phase organique avec l'eau contenant du dioxyde de carbone.

2. Procédé selon la revendication 1, caractérisé en ce que l'on travaille dans l'extraction et/ou réextraction sous une pression de dioxyde de carbone.

3. Procédé selon la revendication 1 ou 2, caractérisé en ce que la pression du dioxyde de carbone est comprise entre 1 et 70 bar.

4. Procédé selon la revendication 3, caractérisé en ce que la température d'extraction et de réextraction est comprise entre le point de solidification de la solution et 150 °C.

5. Procédé selon une ou plusieurs des revendications 1 à 4, caractérisé en ce que l'on choisit le solvant organique parmi les alcools, les esters, les cétones et les éthers.

6. Procédé selon une ou plusieurs des revendications 1 à 5, caractérisé en ce que l'on met en oeuvre ce procédé en continu.

7. Procédé selon une ou plusieurs des revendications 1 à 5, caractérisé en ce que l'on utilise des sels de alcalins ou de métaux alcalino-terreux des composés organiques acides à libérer.

8. Procédé selon une ou plusieurs des revendications 1 à 7, caractérisé en ce que l'on effectue une séparation sélective des composés organiques acides par extraction.

9. Procédé selon une ou plusieurs des revendications 1 à 8, caractérisé en ce que l'on utilise un sel ou des sels de l'acide 6-hydroxy-2-napthalènecarboxylique.
